Europäisches Patentamt

European Patent Office

Office européen des brevets

(11) Veröffentlichungsnummer: **0 167 840**

**A1**

(12) # EUROPÄISCHE PATENTANMELDUNG

(21) Anmeldenummer: **85107038.3**

(22) Anmeldetag: **07.06.85**

(51) Int. Cl.⁴: **A 23 L 1/337**
**A 23 L 1/304, A 23 L 1/30**

(30) Priorität: **09.06.84 DE 3421644**

(43) Veröffentlichungstag der Anmeldung:
**15.01.86 Patentblatt 86/3**

(84) Benannte Vertragsstaaten:
**AT BE CH DE FR GB IT LI LU NL SE**

(71) Anmelder: **Hau, Richard**
**Rheinbadstrasse 6**
**D-7880 Bad Säckingen(DE)**

(72) Erfinder: **Hau, Richard**
**Rheinbadstrasse 6**
**D-7880 Bad Säckingen(DE)**

(74) Vertreter: **Sternagel, Hans-Günther, Dr. et al,**
**Patentanwälte Dr. M. Hann Dr. H.-G. Sternagel**
**Marburger Strasse 38**
**D-6300 Giessen(DE)**

(54) **Diätetische Zusammensetzung.**

(57) Diätetische Zusammensetzung, die Vitamin E und Selen-verbindungen auf der Mikroalge Spirulina als Trägermaterial enthält, wobei Selen sowohl in gebundener Form als auch in Form zugesetzter Selenverbindung in Mengen von 20 mg bis 200 mg Selen/Kg Spirulina und Vitamin E in Form von α-Tocopherolacetat in mengen von 1 g bis 25 g/Kg Spirulina vorhanden sind. Vorzugsweise enthält eine Einzeldosis von etwa 400 mg Spirulina 110-500 µg $Na_2SeO_3$ oder entspre-chende Mengen anderer organischer Selenverbindungen und 8-15 mg Vitamin E und kann in Pulverform, Tabletten-form vorliegen oder in einer Hartgelatinekapsel eingeschlos-sen sein.

Die Erfindung richtet sich auf eine diätetische Zusammensetzung, mit der dem menschlichen Organismus das Spurenelement Selen in einer ausreichenden, jedoch nicht toxischen Menge zusammen mit Vitamin E in resorbierbarer Form
auf oralem Wege zugeführt werden kann, insbesondere auf
ein neues Trägermaterial für diesen Zweck.

Alan Lewis beschreibt in seinem Buch - Selen - , Semmelweiß-Verlag 1983, das Vitamin E und Selen eine gemeinsame synergistische Wirkung auf den menschlichen Organismus und die Gesundheit ausüben. Es wird angenommen, daß
sich diese synergistische Wirkung auf ein Enzym konzentriert, das Teil eines anderen spezifischen Enzyms, der
Gluthathionperoxidase ist. Selen ist ein wichtiges, vom
Körper benötigtes essentielles Spurenelement. Zunehmend
stellt sich aufgrund wissenschaftlicher Untersuchungen
heraus, daß die Selenversorgung mit den heute üblichen
Nahrungsmitteln nur die untere Grenze der vom Menschen
benötigten Menge erreicht bzw. zu einem erheblichen
Prozentsatz darunter liegt. In der Literatur werden tägliche Grundbedarfswerte zwischen 80 und 200 µg genannt.
Nach Auffassung anderer Wissenschaftler beträgt der tägliche Selenbedarf eines Erwachsenen 250 - 300 µg.

Aufgabe der Erfindung ist es, eine diätetische Zusammensetzung zu schaffen, mit der auf oralem Wege dem menschlichen Organismus eine ausreichende Menge an Selen in Kombination mit Vitamin E zugeführt werden kann, jedoch eine Zufuhr toxischer Mengen an Selen praktisch ausgeschlossen ist.

Diese Aufgabe wird gelöst durch die Verwendung der getrockneten Mikroalge Spirulina platensis als Trägermaterial zum Herstellen einer diätetischen Zusammensetzung in Pulver oder Tablettenform, die Vitamin E in Form von $\alpha$-Tocopherolacetat in einer Menge von 1 g bis 25 g/kg Spirulina und Selenverbindungen in einer Menge von 20 mg bis 200 mg Se/kg Spirulina enthält.

In den Unteransprüchen sind bevorzugte Ausführungsformen der Erfindung beschrieben.

Das erfindungsgemäße Diätetikum ermöglicht es auch als Nahrungszusatz dem menschlichen Körper Selen in ausreichender Menge zuzuführen, wobei wegen der synergistischen Wirkung noch Vitamin E ($\alpha$-Tocopherolacetat) sowie als Trägermaterial die Mikroalge Spirulina verwendet wird. Die Verwendung von Spirulina platensis als Trägermaterial ermöglicht eine orale Verabreichung von Selen. Dabei kann Selen sowohl in gebundener Form durch Aufnahme von Selenverbindungen aus dem Wachstumsmedium beim Wachstum der Mikroalge als auch in Form von dem trockenen Trägermaterial zugesetzten Selenverbindungen vorhanden sein. In gebundener Form handelt es sich um organische Selenverbindungen. Werden Selenverbindungen dem trockenen Trägermaterial zugemischt, können sowohl organische als auch anorganische Selenverbindungen zugesetzt werden. $Na_2SeO_3$ ist die bevorzugte anorganische Selenverbindung. Die Kombination mit dem Trägermaterial Spirulina ermöglicht es, dem Körper anorganische Selenverbindungen zuzuführen, ohne daß praktisch toxische Mengen an Selen dem Körper zugeführt werden.

Der Vorteil der anorganischen Selenverbindung gegenüber den an sich besser resorbierbaren organischen Selenverbindungen besteht darin, daß die organischen Selenverbindungen vom Körper häufig bei der Proteinsynthese anstelle schwefelhaltiger Aminosäuren eingebaut werden und damit nicht in der vom Körper benötiden Form zur Verfügung stehen .

Die als Trägermaterial verwendete Mikroalge Spirulina ist leicht verdaulich und enthält außerdem viele wertvolle Nährstoffe, so daß es sich nicht um einen unverdaulichen Trägerstoff handelt, sondern das Diätetikum einen Teil des täglichen Nährstoffbedarfes dem Organismus zuführt. Aufgrund dieser Koppelung an einen natürlichen Nährstoff wir die Aufnahme und Verwertung im Organismus verbessert.

Ein besonderer Vorteil des erfindungsgemäßen Trägermaterials besteht darin, daß die Mikroalge Spirulina einen hohen Eiweißgehalt aufweist, so daß eine eventuelle Toxizität anorganischer Selenverbindungen durch den hohen Proteingehalt kompensiert wird.

Aus epidemiologischen Untersuchungen läßt sich ableiten, daß die Zufuhr von 0,2 mg Se/Kg Körpergewicht geringe Schäden wie vorübergehende Übelkeit verursachen kann.

Der $LD_{50}$ Wert von Natriumselenit bei intramuskulärer Injektion beträgt für Kaninchen 25 mg Se/kg Körpergewicht. Bei oraler Applikation erhöht sich diese letale Dosis auf 1,0 mg Se/Kg Körpergewicht.

Um bei der erfindungsgemäßen Zusammensetzung eine toxische Wirkung beim Menschen zu erreichen, müßte bei einem durchschnittlichen Körpergewicht ca. 250 g der erfindungsgemäßen Zusammensetzung innerhalb einer Stunde oral dem Körper zugeführt werden, um an die toxische Barriere zu gelangen. Eine solche Zufuhr ist jedoch praktisch ausgeschlossen. In diesem Zusammenhang ist es noch wesentlich, daß durch die Kombination mit dem stark proteinhaltigen Trägermaterial die Toxizität von Selen zusätzlich gesenkt wird.

Eine geeignete Einzeldosis enthält etwa 300 mg bis 450 mg, vorzugsweise etwa 400 mg Spirulina. Die Einzeldosis wird in Pulverform, Tablettenform ausgebildet oder in eine Hartgelatinekapsel eingeschlossen.

Um den Tagesbedarf des menschlichen Körpers sowohl von Selen als auch von Vitamin E zu decken, enthält das Trägermaterial Spirulina 20 mg bis 200 mg, vorzugsweise 50 mg bis 150 mg Selen pro Kg Trägermaterial. Ganz besonders bevorzugt ist eine Dosierung von 75 mg Selen pro Kg Spirulina. Vitamin E ist als $\alpha$-Tocopherolacetat in Mengen von 1 g bis 25 g/Kg Spirulina, vorzugsweise 5 g bis 10 g/Kg Spirulina anwesend. Ganz besonders bevorzugt ist eine Einzeldosis von 400 mg Spirulina mit 3 mg $\alpha$-Tocopherolacetat.

Die erfindungsgemäße diätetische Zusammensetzung wird aus dem sprühgetrockneten oder gefriergetrocknetem Trägermaterial Mikroalge Spirulina hergestellt. Dabei kann der natürliche Gehalt an Selen von Spirulina dadurch erhöht sein, daß dem Wachstumsmedium beim Züchten der Mikroalge Selenverbindungen vorzugsweise $Na_2SeO_3$ zugesetzt wurden. Bei Anwesenheit von Selenverbindungen im wässrigen Wachstumsmedium werden diese in erheblichen Mengen beim Wachstum der Mikroalge aufgenommen und gebunden.

Verwendet man Selenkonzentrationen von 1-3 Mol Selen pro
100 l in Form einer wasserlöslichen Selenverbindung
(selenige Säure) Wachstumsmedium, so nimmt die Microalge
Spirulina Selen auf.
Das Wachstum der Algen erfolgte bei pH-Werten zwischen
9.0 und 11.0 unter den für das Spirulina-Wachstum üblichen
Temperaturen und Lichtverhältnissen.
Die Probennahme erfolgte nach 14, 21, 28 und 35 Tagen.
Der Selegengehalt wurde an getrockneten Microalgen bestimmt.

**Selenaufnahme von Spirulina-Algen in Abhängigkeit von Wachstumszeit und molarer Selenkonzentration.**
(Algenpulver getrocknet)

| Selenkonzentration mol/100 l | Selengehalt von Spirulina Algen nach | | | |
|---|---|---|---|---|
| | 14 Tagen mg Se/kg | 21 Tagen mg Se/kg | 28 Tagen mg Se/kg | 35 Tagen mg Se/kg |
| 1,0 | 3,8 +/- 0,2 | 13,6 +/- 0,4 | 13,4 +/- 0,2 | 13,7 +/- 0,2 |
| 1,5 | 4,4 +/- 0,2 | 12,5 +/- 0,7 | 14,2 +/- 0,4 | 13,5 +/- 0,6 |
| 2,0 | 4,1 +/- 0,1 | 13,3 +/- 0,8 | 14,0 +/- 0,3 | 14,3 +/- 0,1 |
| 2,5 | 5,1 +/- 0,3 | 13,8 +/- 0,4 | 13,6 +/- 0,5 | 13,7 +/- 0,5 |
| 3,0 | 4,3 +/- 0,2 | 13,6 +/- 0,6 | 13,8 +/- 0,3 | 13,5 +/- 0,4 |

Die angegebenen Werte errechnen sich aus einem statistischen
Mittel von je sieben unabhängig voneinander ermittelten Einzelwerten.

Die Regressionskoeffizienten liegen unter R 0,9889. Die Werte
sind somit statistisch gesichert.

Bei schonenderer Trocknung, beispielsweise Gefriertrocknen,
liegen die in den getrockneten Proben gefundenen Selenwerte
um den Faktor $10^3$ höher.

Dies läßt den Schluß zu, daß sich beim Trocknen ein Teil der
Selenverbindungen verflüchtigt und die Trockenbedingungen
deshalb sorgfältig überwacht werden müssen, wenn durch entsprechend niedrige Konzentrationen von Selenverbindungen
im Wachstumsmedium der gewünschte Endgehalt an Selen im getrockneten Material direkt erhalten werden soll.

Um den Gehalt

zu standardisieren, können der getrockneten Mikroalge noch zusätzlich Selenverbindungen bei der Herstellung der erfindungsgemäßen Zusammensetzung zugemischt werden. Die Zusatzmenge richtet sich nach dem natürlichen Gehalt an Selen und wird so bemessen, daß der gewünschte Selengehalt erreicht wird.

Durch Zusatz von $\alpha$-Tocopherolacetat zum Wachstumsmedium läßt sich der natürliche Gehalt an Vitamin E von Spirulina ebenfalls erhöhen, so daß zum Einstellen des gewünschten Gehalts an Vitamin E dieses beim Herstellen der erfindungsgemäßen Zusammensetzung gegebenenfalls nicht oder nur in geringerer Menge zugemischt wird.

Die Herstellung der erfindungsgemäßen Zusammensetzung bzw. des Diätetikums erfolgt durch Mischen der Bestandteile, Spirulina Mikroalgenpulver, $Na_2SeO_3$ und Vitamin E. Vorzugsweise wird noch feinteilige Kieselsäure, beispielsweise feinteilige pyrogene Kieselsäure zugemischt. Dieser Zusatz erleichtert die Weiterverarbeitung zu Einzeldosen. Das entstehende homogen gemischte Pulver kann entweder direkt als Nahrungsmittelzusatz verwendet werden oder zu Einzeldosen zur leichteren Einnahme aufbereitet werden. Geeignete Einzeldosen können pulverförmig, als Tablette oder Dragee ausgebildet sein. Einschließen in Hartgelatinekapseln ist eine weitere Form der Einzeldosis.

Beispiel 1

1oo Kg sprühgetrocknete oder gefriergetrocknete Mikroalge
Spirulina in Pulverform werden mit 18,25 g $Na_2SeO_3$ und
5oo g Vitamin E ($\alpha$-Tocopherolacetat)in einem Pulvermischer
homogen vermischt.

Das entstandene homogene Pulver dient als Nahrungszusatz,
wobei die Dosierung so gewählt wird, daß bei üblicher
Menge an Tagesaufnahme des Nahrungsmittels die erwünschte
Tagesdosis von 5o - 2oo ug Selen mit aufgenommen wird.

Beispiel 2

4o Kg Mikroalge Spirulina in Pulverform werden mit 7,3 g
$Na_2SeO_3$ und 2oo g $\alpha$-Tocopherolacetat und 2oo g pyrogener
Kieselsäure in einem Pulvermischer homogen vermischt und
das erhaltene Pulver zu Einzeldosen in Tablettenform von
4oo mg Gewicht verpreßt.

Beispiel 3

1oo Kg Mikroalge Spirulina in Pulverform werden mit 5oo g
$\alpha$-Tocopherolacetat und 18,25 g $Na_2SeO_3$ in einem Pulvermischer homogen vermischt und das Pulver in Hartgelatinekapseln zu Einzeldosen abgefüllt, wobei die Einzeldosis
3o3 mg Pulvergemisch enthält.

Beispiel 4

Dem Wachstumsmedium (Wasser) für Spirulina wird neben den
üblichen Zusätzen für das Wachstum $Na_2SeO_3$ zugesetzt, so
daß die Mikroalge Spirulina in sprühgetrockneter Form
einen Selengehalt von 7,5 mg Se/Kg Spirulina aufweist.
Zur Herstellung der erfindungsgemäßen Zusammensetzung

werden 5 - 15 g $\alpha$ -Tocopherol/Kg Spirulina zugemischt.

Beispiel 5

Das Wachstumsmedium (Wasser) für Spirulina wird neben den üblichen Zusätzen $\alpha$ -Tocopherol zugesetzt, so daß das sprühgetrocknete Spirulinapulver 15 g Vitamin E/Kg Spirulina enthält. Zur Herstellung der erfindungsgemäßen Zusammensetzung wird feingemahlenes $Na_2SeO_3$ in einer Menge von 11o mg bis 328,5 mg/Kg Spirulina zugemischt.

Beispiel 6

Dem wässrigen Wachstumsmedium für die Mikroalge Spirulina werden neben üblichen Zusätzen sowohl $Na_2SeO_3$ als auch $\alpha$ -Tocopherol zugesetzt, so daß das sprühgetrocknete Pulver bis zu 6o mg Se und 15 g Vitamin E pro Kg getrocknete Mikroalge enthält. Dem sprühgetrockneten Pulver kann für die Weiterverarbeitung zu Einzeldosen noch feinteilige pyrogene Kieselsäure zugemischt werden, es kann jedoch auch direkt als erfindungsgemäße Zusammensetzung verwendet werden.

Ein besonderer Vorteil der erfindungsgemäßen Zusammensetzung besteht darin, daß Selen in gut resorbierbarer Form dem menschlichen Organismus angeboten und von diesem auch zu einem erheblichen Anteil aufgenommen wird.

Sechs Einzeldosen zu je 400 mg, die zusammen 186 µg Se enthalten, wurden insgesamt 7 Versuchspersonen über den Tag verteilt verabreicht und eine Stoffwechselbilanz des Selens erstellt. Der Selenspiegel im Urin der Testpersonen vor Verabreichung der erfindungsgemäßen Zusammensetzung betrug im Durchschnitt 8 µg Se/1 Flüssig-

keit, in der Faeces lag der Wert unter der Nachweisgrenze.

Nach Verabreichung der Tagesdosis wurden folgende
Mittelwerte ermittelt:

a) Urin nach 12 h          11 µg Se/l

b) Urin nach 24 h          20 µg Se/l

c) Faeces nach 12 h        kein Selen nachweisbar

d) Faeces nach 24 h        82 µg Se/kg

Daraus ergibt sich als Gesamtbilanz:

zugeführte Selenmenge   186 µg

vom Organismus abgegebene Selenmenge       113 µg (31 µg über Urin,
                        _____ 82 µg über Faeces)

vom Organismus aufgenommene Selenmenge       73 µg

Geht man davon aus, daß auch die über den Urin ausgeschiedene Selenmenge von 31 µg während 24 h dem Stoffwechsel zur dauerhaften Aufnahme zur Verfügung stand,
also im Mittel insgesamt 104 µg Se den Metabolismus
der Versuchspersonen passierten, ergibt sich, daß die
erfindungsgemäße Zusammensetzung besonders geeignet
ist, den in der Literatur beschriebenen Tagesbedarf
an Selen zu decken.

Patentansprüche

1. Verwendung der getrockneten Mikroalge Spirulina platensis als Trägermaterial zum Herstellen einer diätetischen Zusammensetzung in Pulver- oder Tablettenform, die Vitamin E in Form von $\alpha$-Tocopherolacetat in einer Menge von 1 g bis 25 g/kg Spirulina und Selenverbindungen in einer Menge von 20 mg bis 200 mg Se/kg Spirulina enthält.

2. Verwendung nach Anspruch 1,
d a d u r c h   g e k e n n z e i c h n e t ,
daß getrocknete Mikroalgen verwendet werden, die organische Selenverbindungen enthalten, die durch Aufnahme von Selenverbindungen aus dem Wachstumsmedium beim Wachstum der Mikroalge Spirulina gebildet werden.

3. Verwendung nach Ansprüchen 1 oder 2,
d a d u r c h   g e k e n n z e i c h n e t ,
daß der Selengehalt in der getrockneten Mikroalge Spirulina durch zugesetztes $Na_2SO_3$ eingestellt ist.

4. Verwendung nach Ansprüchen 1 bis 3,
d a d u r c h   g e k e n z e i c h n e t ,
daß das Trägermaterial noch 25 bis 150 g/kg Spirulina feinteilige Kieselsäure enthält.

## Europäisches Patentamt

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | DE-A-2 124 972  (AMERICAN GENERAL ENTERPRISES INC.) * Ansprüche 1,9 * | | A 23 L  1/337 A 23 L  1/304 A 23 L  1/30 |
| A | DE-A-1 767 269  (INSTITUT FRANCAIS DU PETROLE DES CARBURANTS ET LUBRIFIANTS) * Beispiel 1, Ansprüche 1,3,6 * | | |
| A | DE-A-1 617 340  (A. BOUCLET) * Ansprüche 1,2 * | | |

RECHERCHIERTE SACHGEBIETE (Int. Cl 4)

A 23 L  1/00

Der vorliegende Recherchenbericht wurde für alle Patentansprüche erstellt.

| Recherchenort BERLIN | Abschlußdatum der Recherche 03-09-1985 | Prüfer SCHULTZE D |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82